(19) Europäisches Patentamt / European Patent Office / Office européen des brevets

(11) **EP 2 525 810 B1**

(12) **EUROPEAN PATENT SPECIFICATION**

(45) Date of publication and mention of the grant of the patent:
**23.09.2015 Bulletin 2015/39**

(21) Application number: **11700459.8**

(22) Date of filing: **21.01.2011**

(51) Int Cl.:
**A61K 38/57** (2006.01)  **C12Q 1/56** (2006.01)
**A61P 33/02** (2006.01)

(86) International application number:
**PCT/EP2011/050838**

(87) International publication number:
**WO 2011/089225 (28.07.2011 Gazette 2011/30)**

(54) **USE OF APROTININ FOR TREATING PARASITIC INFECTIONS AND PROGNOSING BOVINE TRYPANOTOLERANCE**

VERWENDUNG VON APROTININ ZUR BEHANDLUNG PARASITÄRER INFEKTIONEN UND ZUR PROGNOSE DER TOLERANZ DER RINDER FÜR TRYPANOSOM

UTILISATION DE L'APROTININE POUR LE TRAITEMENT DES INFECTIONS PARASITAIRES ET POUR LE PRONOSTIC DE LA TRYPANOTOLERANCE BOVINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.01.2010 FR 1050430**

(43) Date of publication of application:
**28.11.2012 Bulletin 2012/48**

(73) Proprietors:
• **Institut De Recherche Pour Le Développement (IRD)**
**13002 Marseille 2 (FR)**
• **Centre de Coopération Internationale en Recherche Agronomique pour le Développement (CIRAD)**
**75016 Paris (FR)**

(72) Inventors:
• **BERTHIER, David**
**F-34300 Agde (FR)**
• **CUNY, Gerard**
**F-34160 Castries (FR)**
• **THEVENON, Sophie**
**F-34830 Jacou (FR)**
• **CHANTAL, Isabelle**
**F-34270 Fontanes (FR)**
• **BOISSIERE, Anne**
**F-34080 Montpellier (FR)**

(74) Representative: **Cabinet Plasseraud**
**52, rue de la Victoire**
**75440 Paris Cedex 09 (FR)**

(56) References cited:
**US-A1- 2009 074 783**

• **CARDIGAN R.A. ET AL.: "Determination of plasma aprotinin levels by functional and immunological assays", BLOOD COAGULATION AND FIBRINOLYSIS, vol. 12, 2001, pages 37-42, XP002594711,**
• **DATABASE BIOSIS [Online] BIOSCIENCES INFORMATION SERVICE, PHILADELPHIA, PA, US; 1990, FULLER A L ET AL: "REDUCTION IN CELL ENTRY OF EIMERIA-TENELLA COCCIDIA SPOROZOITES BY PROTEASE INHIBITORS AND PARTIAL CHARACTERIZATION OF PROTEOLYTIC ACTIVITY ASSOCIATED WITH INTACT SPOROZOITES AND MEROZOITES", XP002594712, Database accession no. PREV199191051186**
• **DATABASE BIOSIS [Online] BIOSCIENCES INFORMATION SERVICE, PHILADELPHIA, PA, US; 1996, FORNEY J R ET AL: "Efficacy of serine protease inhibitors against Cryptosporidium parvum infection in a bovine fallopian tube epithelial cell culture system", XP002594713, Database accession no. PREV199699156879**

- CHOUDHURY R. ET AL.: "Identification, purification and characterization of a secretory serine protease in an Indian strain of Leishmania donovani.", MOL. CELL. BIOCHEM., vol. 320, 2009, pages 1-14, XP002594714,
- PIGUET P.F. ET AL.: "delayed mortality and attenuated thrombocytopenia associated with severe malaria in urokinase- and urokinase receptor-deficient mice.", INFECTION AND IMMUNITY, vol. 68, no. 7, July 2000 (2000-07), pages 3822-3829, XP002594715,
- VERDOT L. ET AL.: "Cystatins up-regulate nitric oxide release from interferon gamma-activated mouse peritoneal macrophages", J. BIOL. CHEM., vol. 271, no. 45, 1996, pages 28077-28081, XP002594716,
- DATABASE BIOSIS [Online] BIOSCIENCES INFORMATION SERVICE, PHILADELPHIA, PA, US; 1983, DEJKRIENGKRAIKHUL P-N ET AL: "REQUIREMENT OF MALARIAL PROTEASE IN THE INVASION OF HUMAN RED CELLS BY MEROZOITES OF PLASMODIUM FALCIPARUM", XP002594717, Database accession no. PREV198376082433

**Description**

**Field of the Invention**

The present invention relates to the use of aprotinin for the treatment of parasitic infections, to fight the pathogenic effects caused by parasites of the *Trypanosomatidae* family. The invention also relates to the use of aprotinin for the prognosis of animal tolerance to these parasitic infections.

**Background of the Invention**

Parasitic diseases are cause for concern both in terms of their impact on public health and their consequences on the economy of developing countries. They are particularly worrying since re-emergence phenomena have been observed in the last twenty years. Thus, in Africa, human trypanosomiasis (HAT) which, at one time, was practically eradicated, and animal trypanosomosis (AAT) currently represent serious problems in the fields of public and animal health and of agricultural economy.

African trypanosomosis is caused by flagellate blood protozoa (trypanosomes) and is essentially transmitted by a hematophagous insect, the glossina or tsetse flies, in which the parasite achieves a more or less complex cyclic evolution before further transmission. In humans, these parasites are the cause of "sleeping sickness" (or HAT). The World Health Organization (WHO) has estimated that, in Africa, there are more than 250 centers of infection with approximately 60 million people exposed and 300,000 subjects infected each year. In many African countries, the prevalence of the disease is currently equivalent to that in the 1930s before the implementation of disease monitoring and screening measures.

Animal trypanosomosis, also referred to as Nagana, is raging in all regions of Africa infested with tsetse flies, *i.e.* in one third of the African continent. The disease affects about 40 countries, and 50 million cattle and 100 million small ruminants are estimated to be exposed. At the present time, animal trypanosomosis remains a major obstacle to the development of livestock farming and agriculture in regions of Sub-Saharan Africa which otherwise offer strong fodder crop and agricultural potential. Indeed, in infected animals, the disease can give rise to delayed growth, significant weight loss, lower fertility, a higher abortion rate, a drop in milk production and/or reduced drawing capabilities, and in the most severe cases, rapid death. Livestock morbidity and mortality in fragile rural economies have disastrous consequences on human nutrition and cause considerable economic losses in many African countries.

Trypanosomosis most frequently progresses in a chronic form - this is the case in human trypanosomosis caused by *Trypanosoma brucei gambiense* (90% of cases reported) found in Central and West Africa, and the majority of cases of animal trypanosomosis (caused by *T. brucei brucei*, *T. congolense* or *T. vivax*). The acute form in humans is caused by *T. brucei rhodesiense* in Eastern and Southern Africa, and represents 10% of reported cases on average. In animals, some *T. congolense* and *T. vivax* strains can cause rapidly progressing severe forms. The symptoms vary and are frequently linked with the trypanosome species and also with host susceptibility. Some cattle breeds *(Bos taurus:* N'Dama) are, for example, much more tolerant to infections than exotic breeds *(Bos indicus:* zebu, and European *Bos taurus*). This trypanotolerance is subject to multiple factors and genetic dependence.

At the present time, control measures are available, but they have proven to be insufficient to control the disease. These methods target the parasite or the vector. Anti-vector controls are conducted at a number of levels: on an individual scale *via* the epicutaneous application of insecticide formulations to livestock or the set-up of attractive baits and on a regional scale *via* aerial distribution. Biological control, particularly the introduction of sterile steste flies males, is also used. Although these methods are effective in restricted areas, they have proven to be insufficient to eradicate tsetse flies on vaster territories. Parasite control consists of administering curative or preventive trypanocidal treatments. For animal African trypanosomosis, the treatments differ according to the parasite species. In ruminants, diminazene acetate (BERENIL® and VERIBEN®) and isomethamidium chloride (SAMORIN® and TRYPANIDIUM®) are the most commonly used compounds, particularly against *T. vivax*, *T. congolense* and *T. brucei*. As no other trypanocidal agent has been introduced onto the market for over thirty years, these medicinal products have been used intensively, but unsuitable use has widely contributed to the appearance of resistant trypanosome strains.

Thus, the development of novel control strategies and novel treatments against animal trypanosomosis which affects not only many African countries but also other parts of the globe such as Latin America and South-East Asia is highly desirable.

**Summary of the Invention**

In their study of trypanotolerance in cattle, the inventors have focused on the genes involved in this phenomenon. They thus identified a serine protease inhibitor, aprotinin, which, in tolerant animals, is over-expressed when the blood parasite level peaks but whose expression in susceptible animals is relatively low and stable. Aprotinin or BPTI (Bovine Pancreatic Trypsin Inhibitor) is a relatively well-known molecule that has been widely used in heart surgery for its anti-inflammatory properties and anti-thrombotic and anti-fibrinolytic properties. However, this molecule of bovine origin has caused allergic reactions, which have been violent in some patients, and which have led Europe and the United States to ban its use in humans.

The inventors have demonstrated, for the first time, that aprotinin has significant anti-parasitic effects not only on trypanosomes but also on leishmania. Indeed, the results they obtained demonstrate not only that *in vitro* parasite mortality is increased by 50% in the presence of aprotinin (see Example 2) but also that aprotinin causes inhibition of more than 50% of the enzyme activity of *in vitro* parasite lysates (see Example 3). Furthermore, aprotinin has a high inhibitory activity on nitric oxide (NO) production in macrophages, which play a decisive role in the response of the host to protozoan infections (see Example 1).

Consequently, a first aspect of the present invention concerns the anti-parasite action of aprotinin, and a second aspect of the invention concerns the role of aprotinin in animal trypanosomosis tolerance phenomena.

More specifically, in a first aspect, the invention relates to the use of aprotinin for treating animal parasitic diseases or infections caused by protozoa of the Trypanosomatidae family, particularly parasitic diseases affecting domesticated mammals. Domesticated animals include in particular livestock, preferably ruminants (cattle, goats and sheep) and swine, and more preferably cattle and swine.

Aprotinin is used for treating animal parasitic diseases or infections caused by flagellate protozoa. The parasitic diseases or infections caused by flagellate protozoa affecting domesticated mammals include, non-exhaustively, lambliasis, trypanosomosis, leishamaniasis and trichomoniasis. In the present invention, aprotinin is used for treating animal parasitic diseases or infections caused by parasites of the *Trypanosomatidae* family, particularly parasites belonging to the *Trypanosoma* genus or *Leishmania* genus.

The *Trypanosoma* genus parasites include, but are not limited to, *T. ambystomae*, *T. avium*, *T. boissoni*, *T. brucei (T. brucei brucei, T. brucei rhodesiensi, T. gambiensis)*, *T. cruzi, T. congolense (T. congolense savannah, T. congolense forest, T. congolense tsavo, T. congolense kilifi)*, *T. equinum, T. eguiperdum, T. megatrvpanum, T. uniforme, T. musculi, T. evansi, T. everetti, T. hosei, T. lewisi, T. godfreyi, T. malophagium, T. paddae, T. parroti, T. percae, T. rangeli, T. rotatorium, T. rugosae, T. sergenti, T. simiae, T. sinipercae, T. suis, T. triglae* and *T. vivax*. The *Trypanosoma* genus parasites that are pathogenic to the cattle include *T. congolense, T. brucei, T. vivax, T. evansi, T. equiperdum, T. theileri* and *T. simiae.* In some preferred embodiments, aprotinin is used in the treatment of parasitic diseases of infections affecting livestock, particularly cattle, and that are caused by *T. congolense, T. brucei* or *T. vivax,* and preferably caused by *T. congolense.*

The *Leishmania* genus parasites include, but are not limited to, *L. aethiopica*, *L. amazonensis*, *L. arabica*, *L. aristidesi, L. deanei, L. donovani, L. enrietti, L. forattinii, L. gorhami, L. herreri, L. hertigi, L. infantum, L. killicki, L. major, L. enriettii, L. mexicana, L. pifanoi, L. tropica, L. (Viannia) braziliensis, L. (Viannia), colombiensis, L. (Viannia) guyanensis, L. (Viannia) lainsoni, L. (Viannia) naiffi, L. (Viannia) panamensis, L. (Viannia) peruviana, L. (Viannia) pifanoi, L. (Viannia) shawi, L. tarentolae, L. tropica, L. turanica*, and *L. venezuelensis*. In particular, *L. infantum*, found in the South of France, is responsible for leishmaniasis in dogs and is also a zoonosis, for which dogs are the reservoir of the human disease. In some preferred embodiments, aprotinin is used in the treatment of parasitic diseases or infections affecting dogs and humans, and caused by *L. infantum* and *L. donovani.*

Described is the use of aprotinin in the treatment of animal parasitic diseases or infections caused by protozoa, affecting cattle. Examples of such parasitic diseases or infections include, but are not limited to, bovine trypanosomosis, bovine lambliasis, bovine trichomoniasis, bovine babesiasis, bovine besnoitiosis, bovine neosporosis, bovine theileriosis, bovine toxoplasmosis, bovine cryptosporidiosis, bovine sarcocystosis and bovine coccidiosis.

The application describes pharmaceutical preparations comprising aprotinin and at least one acceptable veterinary vehicle or excipient. The pharmaceutical preparations are intended to be used for treating an animal parasitic disease or infection, as described above. In some embodiments, the pharmaceutical preparations further comprise at least one additional pharmaceutical active ingredient.

In a further alternative embodiment of the first aspect, the invention relates to the use of aprotinin for producing a medicinal product for treating animal parasitic diseases or infections as described above.

In a further alternative embodiment, the disclosure relates to a method for treating an animal parasitic disease or infection as described above including a step for administering a therapeutically effective quantity of aprotinin to an infected animal.

In a second aspect, the invention relates to aprotinin for its use as a biomarker of bovine trypanotolerance.

In an alternative embodiment of this second aspect, the invention relates to an *in vitro* method for the prognosis of trypanosomosis infections in cattle. The method generally includes a step for determining the quantity of aprotinin in a biological sample obtained from a bovine animal. The biological sample may be any bovine biological fluid or tissue known to contain aprotinin. Preferably, the biological sample is blood or serum sampled from the bovine animal under test. Determining the quantity of aprotinin in the biological sample may be performed using any suitable protein quantification method, for example an immunoassay, such as an ELISA test.

In some preferred embodiments, the quantity of aprotinin determined in the method according to the invention is compared to the mean quantity of aprotinin in a trypanotolerant bovine animal and/or the mean quantity of aprotinin in a susceptible bovine animal. Preferably, the bovine animal being tested and the control bovine animal (trypanotolerant or susceptible) are the same breed, and optionally of same age and same sex. Comparing the quantities of aprotinin allows for the determination of whether the bovine animal tested is tolerant or susceptible to trypanosomosis infections. This method may be used, for example, to select the most tolerant animals in order to distribute the tolerant characteristics throughout a herd.

In a further alternative embodiment, the disclosure relates to a bovine trypanotolerance prognostic kit. The kit comprises a specific antibody for aprotinin, a reagent for detecting an antibody-aprotinin complex formed between the antibody and aprotinin contained in a biological sample, and instructions for carrying out a prognostic method according to the invention.

These and other objects, advantages and features of the present invention will become apparent to those of ordinary skill in the art having read the following detailed description of the preferred embodiments.

**Brief Description of the Drawing**

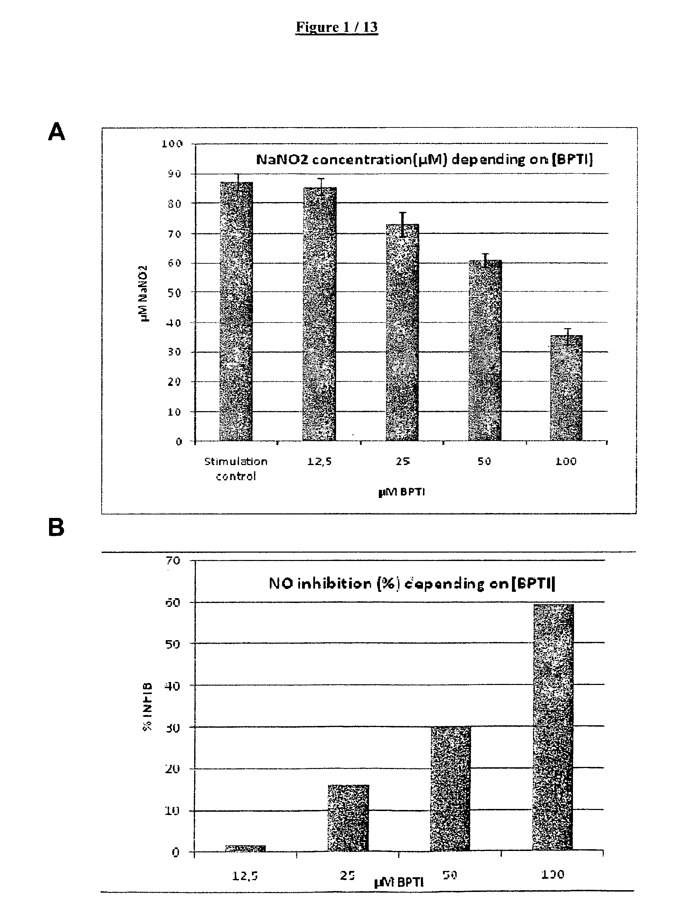

**Figure 1** is a set of two graphs showing the inhibitory effect of BPTI *(i.e.* aprotinin) on the production of NO by macrophages of the murine J774.2 cell line. (**A**) shows the variation of the quantity of NO produced as a function of BPTI concentration, and (**B**) shows the percentage of inhibition in NO production as a function of BPTI concentration.

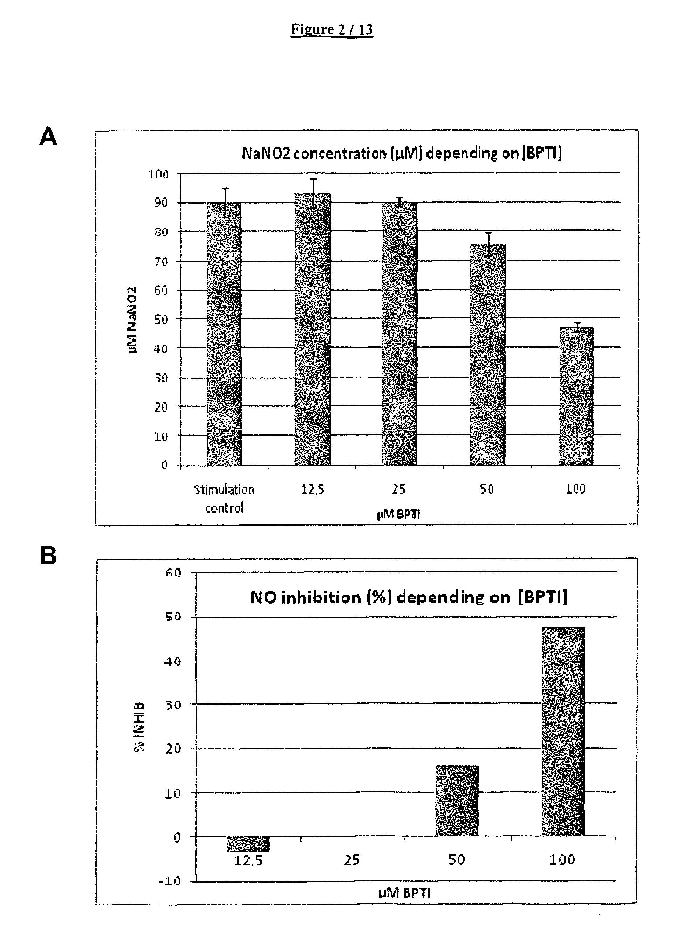

**Figure 2** is a set of two graphs showing the inhibitory effect of BPTI on the production of NO by macrophages of the murine RAW 264.7 cell line. (**A**) shows the variation of the quantity of NO produced as a function of BPTI concentration, and (**B**) shows the percentage of inhibition in NO production as a function of BPTI concentration.

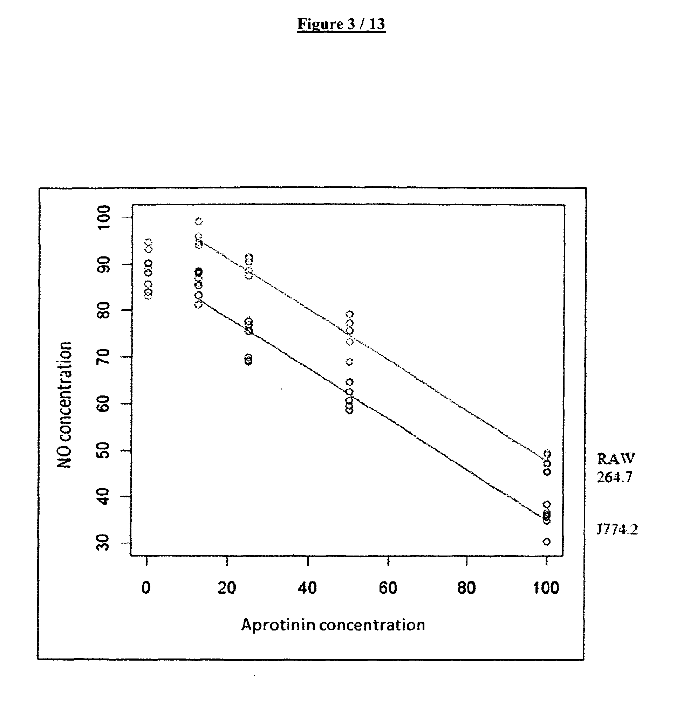

**Figure 3** is a graph showing a comparison of the inhibitory effect of BPTI on the production of NO by macrophages of the J774.2 cell line and of the RAW 264.7 cell line using a model described in Example 1.

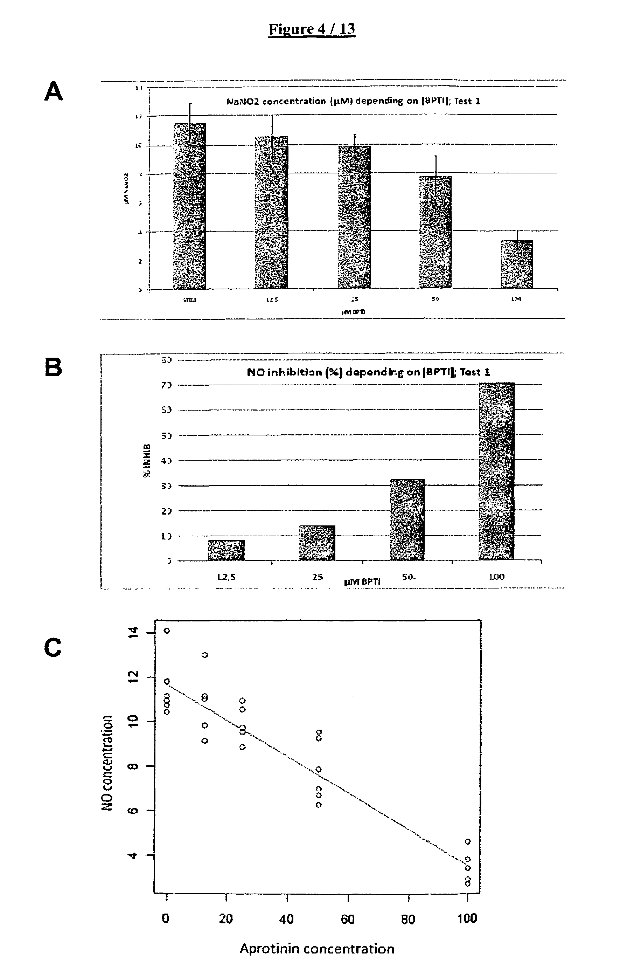

**Figure 4** shows the inhibitory effect of BPTI on the production of NO by bovine macrophages. (**A**) shows the variation of the quantity of NO produced as a function of BPTI concentration, (**B**) shows the percentage of inhibition in NO production as a function of BPTI concentration, and (C) shows an analysis of the results obtained using a model described in Example 1.

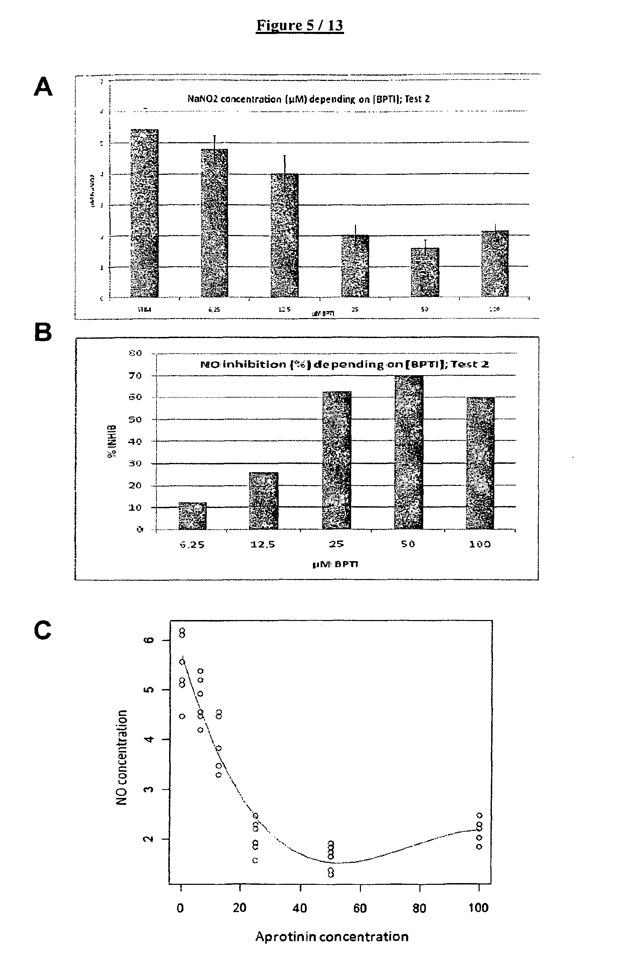

**Figure 5** shows the inhibitory effect of BPTI on the production of NO by bovine macrophages. (**A**) shows the variation of the quantity of NO produced as a function of BPTI concentration, (**B**) shows the percentage of inhibition in NO production as a function of BPTI concentration, and (**C**) shows an analysis of the results obtained using a model described in Example 1.

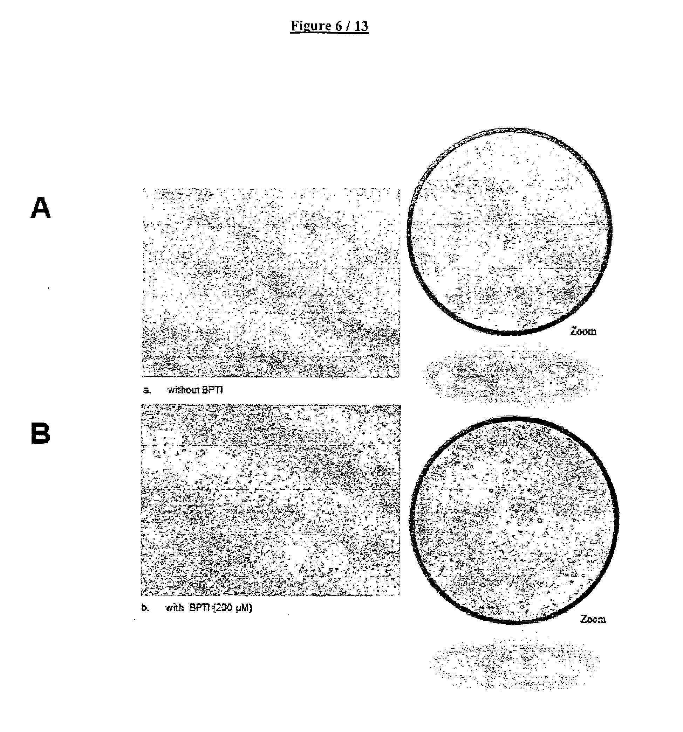

**Figure 6** shows BPTI incidence on *Trypanosoma congolense* IL1180 cultured *in vitro.* (**A**) shows, as a control, a parasite culture observed by microscopy after 24 hours without treatment. (**B**) shows a parasite culture at 24 hours after addition of 200 μM of BPTI.

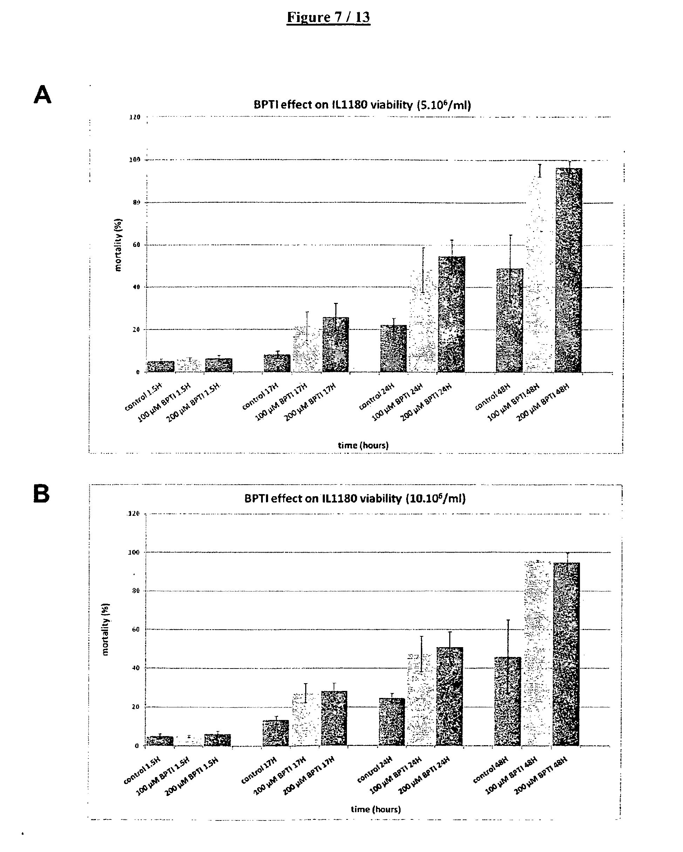

**Figure 7** is a set of two graphs showing the effects of BPTI on *Trypanosoma congolense* IL1180 viability. (**A**) shows the percentage of global mortality *(i.e.,* with no correction for the natural mortality of the control sample) of *Trypanosoma congolense* Tc. IL118 ($5x10^6$) cultured *in vitro*, induced by adding BPTI (100 μM or 200 μM) measured at different times (1.5 hours, 17 h, 24 h and 48 h) after addition of BPTI. (**B**) shows the percentage of mortality *(i.e.,* after correction for the natural mortality of the control sample) of *Trypanosoma congolense* Tc. IL118 ($10x10^6$) cultured *in vitro* induced by adding BPTI (100 μM or 200 μM) measured at different times (1.5 hours, 17 h, 24 h and 48 h) after addition of BPTI.

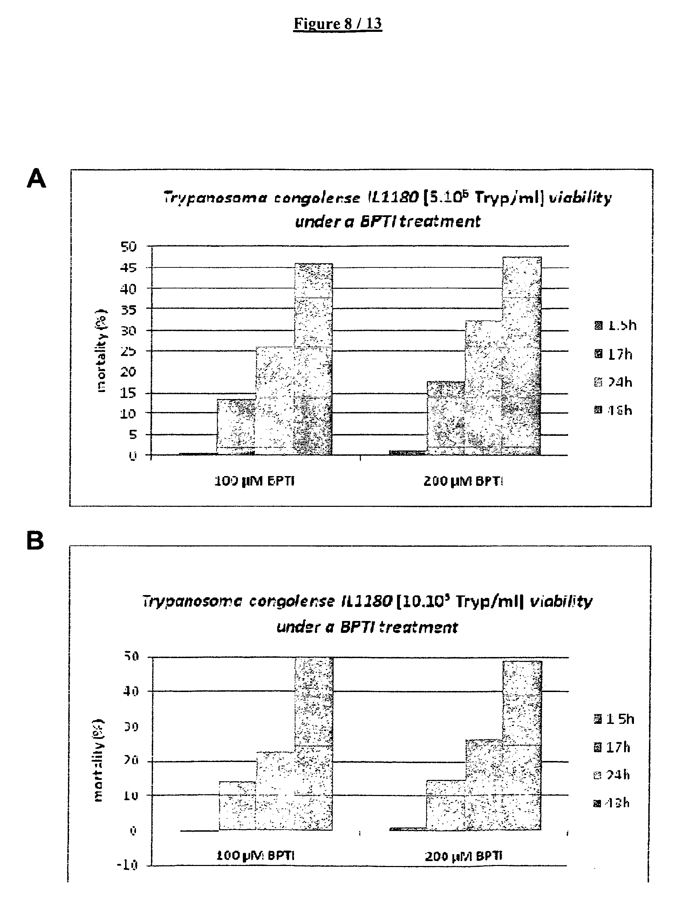

**Figure 8** is a set of two graphs showing the effects of BPTI on *Trypanosoma congolense* IL1180 viability. The graphs show the actual percentage of mortality *(i.e.,* after correction for the natural mortality of the control sample) measured at different times after addition of 100 μM or 200 μM of BPTI in $5x10^6$ (**A**) and $10x10^6$(**A**) *Tiypanosoma congolense* Tc. IL118 cultured *in vitro.*

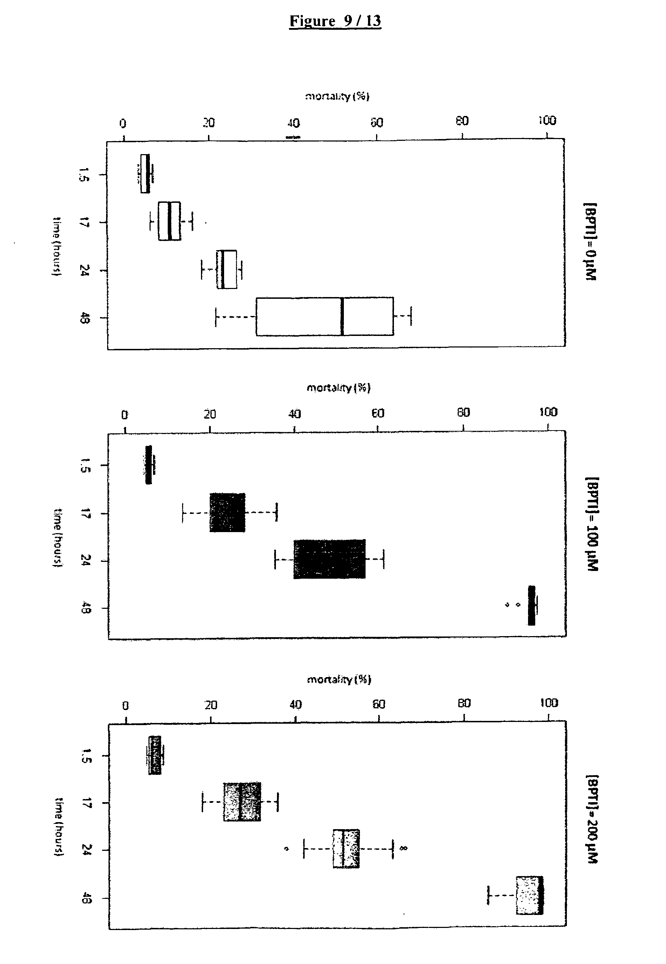

**Figure 9** is a set of three graphs showing the interaction of BPTI concentration (0 μM, 100 μM, 200 μM) and time

of measurement (1.5 hours, 17 h, 24 h and 48 h) on the mortality of trypanosomes, as studied using a model described in Example 2.

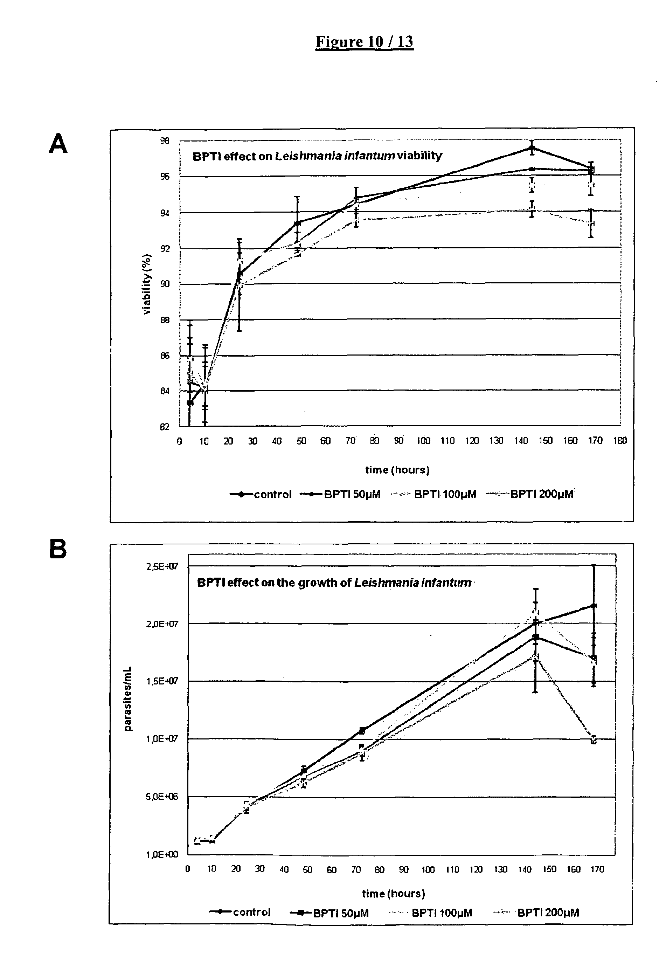

**Figure 10** is a set of two graphs showing the incidence of several concentrations of BPTI on the viability and the growth of a *Leishmania infantum strain* cultured *in-vitro*. (**A**) shows the viability percentage of *Leishmania infantum* cultured without (control) or with 50 µM, 100 µM and 200 µM of BPTI as a function of time after addition of BPTI. (**B**) shows the growth (number of parasites/ml) of *Leishmania infantum* cultured without (control) or with 50 µM, 100 µM and 200 µM of BPTI, as a function of time after addition of BPTI.

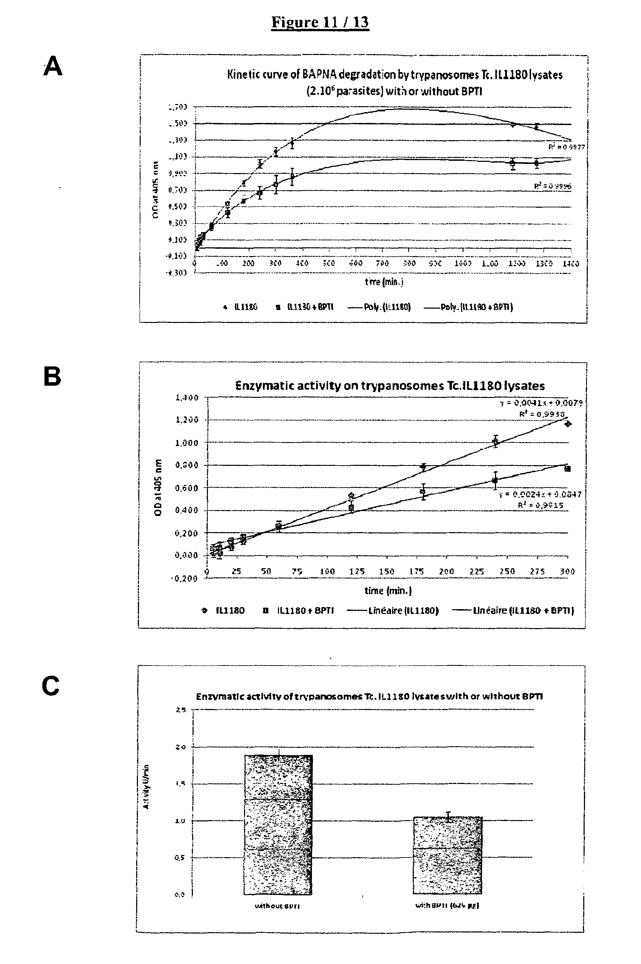

**Figure 11** is a set of three graphs showing the effects of BPTI on the BAPNA degradation induced by *Trypanosoma congolense* IL1180 lysates in the absence (control) or in the presence of BPTI. (**A**) shows the kinetic curve of BAPNA degradation induced by the enzymatic activity of Tc. IL1180 trypanosomes lysates ($2\times10^6$) treated or not (control) with 625 µg of BPTI. (**B**) shows the linear segment of the kinetic curve of BAPNA degradation induced by the enzymatic activity of Tc. IL1180 trypanosomes lysates ($2\times10^6$). (**C**) shows the enzymatic activity of *Trypanosoma congolense* IL1180 lysates treated or not (control) with 625 µg of BPTI.

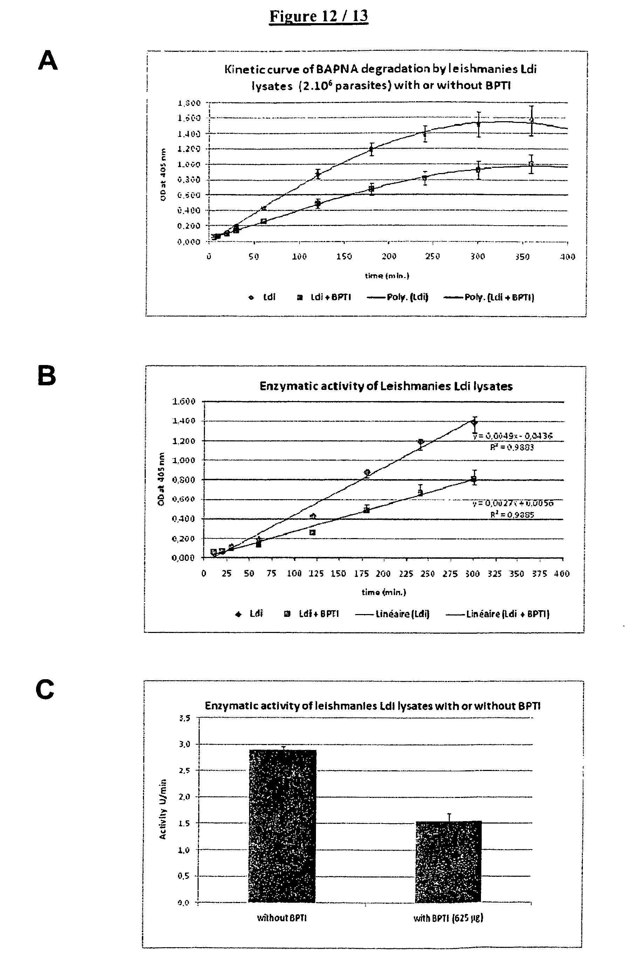

**Figure 12** is a set of three graphs showing the effects of BPTI on the BAPNA degradation induced by *Leishmania donovani infantum (Ldi)* lysates in the absence (control) or in the presence of BPTI. (**A**) shows the kinetic curve of BAPNA degradation induced by the enzymatic activity of the Ldi lysates ($2\times10^6$) treated or not (control) with 625 µg of BPTI. (**B**) shows the linear segment of the kinetic curve of BAPNA degradation induced by the enzymatic activity of the Ldi lysates ($2\times10^6$). (**C**) shows the enzymatic activity of the *Leishmania donovani infantum (Ldi)* lysates treated or not (control) with 625 µg of BPTI.

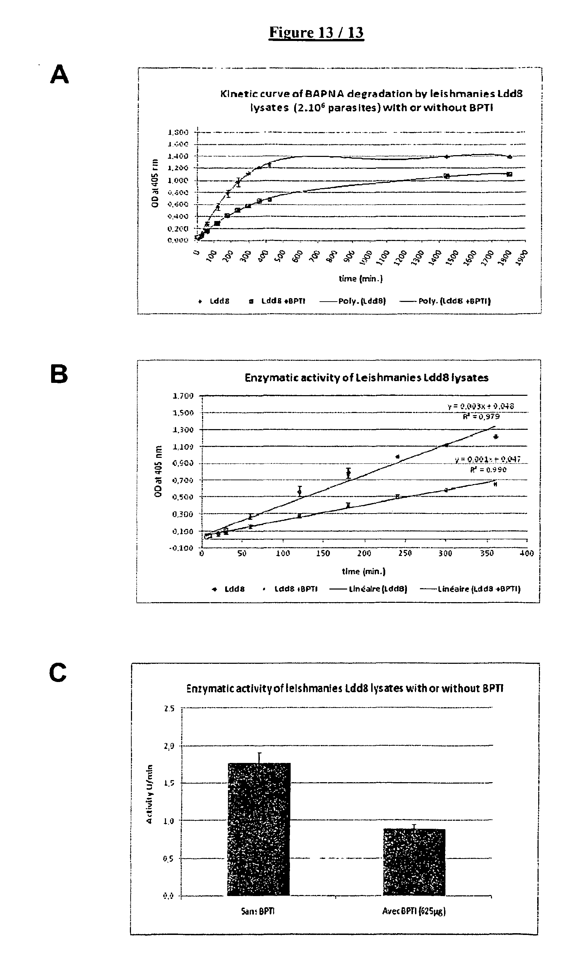

**Figure 13** is a set of three graphs showing the effects of BPTI on the BAPNA degradation induced by *Leishmania donovani donovani (Ldd8)* lysates in the absence (control) or in the presence of BPTI. (**A**) shows the kinetic curve of BAPNA degradation induced by the enzymatic activity of the Ldd8 lysates ($2\times10^6$) treated or not (control) with 625 µg of BPTI. (**B**) shows the linear segment of the kinetic curve of BAPNA degradation induced by the enzymatic activity of the Ldd8 lysates ($2\times10^6$). (**C**) shows the Enzymatic activity of the *Leishmania donovani infantum (Ldi)* lysates treated or not (control) with 625 µg of BPTI.

## Detailed Description of Certain Preferred Embodiments

The present invention generally relates to novel uses of aprotinin in relation with its anti-parasitic action and its role in the trypanotolerance phenomenon in cattle.

## I - Aprotinin as an anti-parasitic agent

As mentioned above, the invention relates to aprotinin for use the treatment of animal parasitic diseases or infections caused by protozoa of the Trypanosomatidae family, affecting mammals, particularly domesticated mammals. In the context of the present invention, the terms "aprotinin", "Bovine Pancreatic Trypsin Inhibitor/IV" and "BPTI" are used interchangeably.

Aprotinin is a Kunitz-type serine protease inhibitor, found in a wide variety of tissues and organs in cattle (pancreas, liver, lungs, parotid gland, spleen, lymph nodes). The proteolytic enzymes known to be inhibited by this molecule include trypsin, kallikrein, chymotrypsin and plasmin. Aprotinin is a monomeric globular polypeptide with a molecular weight of approximately 6512 Daltons, containing 58 amino acids and three disulphide bridges binding the cysteine residues situated in positions 5 and 55, 14 and 38, and 30 and 51 (Kassel et al., Biochem. Biophys. Res. Commun., 1965, 18: 255-258; Kassel et al., Biochem. Biophys. Res. Commun., 1965, 20: 463-468). The gene coding for BPTI has been identified and sequenced (NCBI Reference Sequence: NM_001001554.2).

Aprotinin is known to have some beneficial properties from a therapeutic point of view. In particular, it has an effective action in haemostatic and fibrinolytic system disorders. Prior to its withdrawal from the market, TRASYLOL®, whose active ingredient is aprotinin isolated from bovine organs, has been used for a long time to reduce bleeding in heart surgery and liver transplants (van Oeveren et al., Ann. Thorac. Surg., 1987, 44: 640-645; Bistrup et al., Lancet I, 1988, 366-367; Royston, J. Cardiothorac. Vasc. Anesth., 1992, 6: 76-100; Porte et al., J. Cardiothorac. Vasc. Anesth., 2004, 18(4 Suppl): 31 S-37S). Aprotinin also has anti-inflammatory properties (Asimakopoulos et al., J. Thorac. Cardiovasc. Surg., 2000, 120(2): 361-369; Mojcik et al., Ann. Thorac. Surg., 2001, 71(2): 745-754; Ascenzi et al., Curr. Protein Pept. Sci., 2003, 4(3): 231-251) and antioxidant properties which result from the fact that aprotinin reduces nitric oxide production by inhibiting the inducible form of nitric oxide synthetase (Bruda et al., Clin. Sci. (London), 1998, 94: 505-509; Venturi et al., Biochem. Biophys. Res. Commun., 1998, 249: 263-265).

For the first time, the present inventors have demonstrated the anti-parasitic activity of aprotinin. In the context of the present invention, the terms "aprotinin", "Bovine Pancreatic Trypsin Inhibitor/IV" and "BPTI" are understood to include the polypeptide having 58 amino acids, the formula and structure of which have been known for a long time (CAS number: 9087-70-1), as well as any native aprotinin molecule of bovine origin (*i.e.* isolated from a bovine animal), and any homologue or variant of aprotinin having similar anti-parasitic properties to those of the known polypeptide having 58 amino acids.

Those skilled in the art will understand that, in the context of the present invention, aprotinin may be prepared using any suitable method, the method used for obtaining aprotinin being not a critical or limiting aspect of the invention. Thus, for example, aprotinin may be extracted from natural sources (bovine tissues or organs such as the lungs, pancreas or parotid glands) or prepared using recombinant methods, and such methods may include chemical modification and/or purification steps.

The aprotinin according to the invention may be administered *per se* or in the form of a pharmaceutical preparation or composition. Consequently, the present invention provides the use of pharmaceutical compositions comprising aprotinin and at least one acceptable veterinary vehicle or excipient. In the context of the invention, the term "acceptable veterinary vehicle or excipient" refers to any medium or additive that does not interfere with the efficacy of the biological activity of the active ingredient (in this instance, aprotinin), and which is not excessively toxic for the mammal treated, at the concentrations at which it is administered.

The pharmaceutical compositions may be administered using any dosage combination and administration route which is effective for obtaining the desired therapeutic effect. The exact quantity to be administered may vary between animal species, animal breeds, and even from one animal to the other within the same species and same breed, for example according to weight and/or age. The quantity to be administered may also vary according to the nature of the parasite species. The administration route (oral, parenteral, cutaneous, transdermal, etc.) may be selected according to the type of parasitic disease or infection, the number of animals to be treated, etc.

The formulation of a pharmaceutical composition may vary according to the administration route and dosage. After formulation with at least one acceptable veterinary vehicle or excipient, a pharmaceutical composition may be in any suitable form for administration to a mammal, for examples, tablets, coated tablets, capsules, powders, syrups, solutions for injection, etc. Those skilled in the art are capable of selecting the most suitable vehicles and excipients for preparing a specific type of formulation. Thus, for example, excipients such as water, 2-3-butanediol, isotonic sodium chloride solution, synthetic mono or diglycerides, and oleic acid are frequently used for the formulation of preparations for injection. Liquid compositions, including emulsions, microemulsions, solutions, suspensions, syrups, etc., may be formulated in the presence of solvents, solubilising agents, emulsifiers, oils, fatty acids and other additives such as suspension agents, preservatives, thickeners, etc. Solid compositions for oral administration may be formulated in the presence of an inert excipient such as sodium citrate, and optionally additives such as binding agents, wetting agents, disintegration agents, absorption accelerators, lubricants, etc.

In some embodiments, a pharmaceutical composition is formulated for immediate release of the active ingredient (*i.e.*, aprotinin). Alternatively, a pharmaceutical composition may be formulated for sustained release of the active ingredient. Numerous strategies are known in the art for inducing sustained release of an active ingredient, such as for example increasing the contact time in the stomach, using pH-sensitive coatings and/or enzyme actions, or bioadhesive coatings adhering to the walls of the stomach and intestine, or using encapsulation systems.

The pharmaceutical compositions may further contain at least one further pharmaceutical active ingredient (*i.e.* in addition to aprotinin). The term "pharmaceutical active ingredient" refers to any compound or substance, the administration of which has a therapeutic effect or a beneficial effect on the health or general condition of a mammal to which it is administered. Thus, a pharmaceutical active ingredient may be active against the disease to be treated by administration of the pharmaceutical composition (*i.e.* a parasitic disease or infection causes by a protozoon); or may be active against a condition associated with the disease to be treated by administration of the pharmaceutical composition; or may increase the availability and/or activity of the aprotinin contained in the pharmaceutical composition.

Examples of pharmaceutical active ingredients which may be contained in a composition include, non-exhaustively, antibiotics, anti-inflammatories, growth stimulants, vitamins, and other anti-parasitic agents. As a general rule, the pharmaceutical active ingredient(s) contained in the pharmaceutical composition are suitable for the mammals for which the pharmaceutical composition is intended.

Also described is a method for treating animal parasitic diseases or infections which comprises a step in which a therapeutically effective quantity of aprotinin or of a pharmaceutical composition described herein is administered to a mammal infected by a pathogenic protozoon. In the context of the present invention, the term "treatment" refers to a method intended to (1) delay or prevent the onset of an animal parasitic disease or infection caused by a protozoon, (2) slow down or stop the progression, worsening or deterioration of the symptoms of the disease, (3) improve the symptoms of the disease, and/or (4) cure the disease. A treatment may be administered before the onset of the disease for a preventive action, or may be administered after the onset of disease for a curative action.

Aprotinin (or a pharmaceutical composition according to the invention) may be used for treating any animal parasitic disease or infection caused by a protozoon of the Trypanosomatidae family, for which the administration og aprotinin results in an improvement of the symptoms of the parasitic disease or infection.

In some preferred embodiments, aprotinin is used for treating parasitic diseases or infection affecting domesticated mammals and caused by flagellate protozoa of the Trypanosomatidae family, *i.e.* single-cell eukaryotes with flagella, which are contractile thread-like organs providing locomotion. Examples of such diseases include, but are not limited to, trypanosomosis (caused by *Trypanosoma* genus parasites), leishmaniasis (caused by *Leishmania* genus parasites).

In the present invention, aprotinin is used for treating animal parasitic diseases or infection caused by parasites of the *Trypanosomatidae* family, particularly parasites belonging to the *Trypanosoma* genus or *Leishmania* genus.

In some preferred embodiments, the parasitic disease or infection to be treated according to the present invention affects domesticated mammals, particularly livestock, *i.e.* cattle, sheep, goats and buffalo. Preferably, aprotinin is used for treating parasitic diseases or infections caused by protozoa of the Trypanosomatidae family, affecting cattle. Indeed, since the aprotinin according to the invention is an endogenous molecule, it is extremely well tolerated by cattle. The parasitic diseases or infections caused by protozoa, affecting cattle include, non-exhaustively, bovine trypanosomosis,

The present invention also includes any homologous molecule with respect to aprotinin of mammalian origin other than bovine, for example sheep, goat, canine, etc., and even human origin, for use in the treatment of parasitic diseases or infections caused by protozoa of the Trypanosomatidae family affecting the corresponding mammals, for example, sheep, goats, dogs, etc. and even humans.

## II - Aprotinin as a marker of bovine trypanotolerance

In a further aspect, the invention relates to aprotinin for use as a biomarker of bovine trypanotolerance. The term "biomarker", as used herein, refers to a substance which is a distinctive indicator of a biological process, biological event and/or biological condition. The inventors demonstrated that, in cattle susceptible to trypanosomosis infections, the expression of the gene encoding aprotinin is relatively low and stable whereas in trypanotolerant animals, the expression of said gene is generally higher and the gene is over-expressed when the blood parasite level peaks.

The invention therefore provides an *in vitro* method for the prognosis of trypanosomosis infections in cattle, *i.e.* to determine whether a bovine animal is trypanosomosis-tolerant or susceptible. This method comprises a step of determining the quantity of aprotinin in a biological sample obtained from a bovine animal. The term "biological sample" refers to any fluid or tissue sample isolated from a bovine animal wherein the presence of aprotinin can be detected (blood, serum, pancreas, liver, lungs, parotid gland, spleen, lymph nodes). The biological sample may be taken from a live bovine animal (in which case the biological sample should be selected from those whose preparation/isolation does not require an invasive procedure, for example via drawing blood) or a dead bovine animal (for example by means of biopsy). The sample may also be a cryopreserved archived tissue sample. The term "biological sample" also comprises any material derived from processing or handling a biological sample. The processing or handling of a biological sample may include filtration, centrifugation, distillation, extraction, concentration, interfering compound inactivation, reagent addition steps, etc. If the method is used for testing the trypanotolerance of a live bovine animal, the biological sample is preferably blood or serum.

In some embodiments, the method further comprises a step wherein the quantity of aprotinin measured in the biological sample from the bovine animal tested is compared to the mean quantity of aprotinin in a biological sample of the same type in a trypanotolerant bovine animal and/or in a bovine animal susceptible to trypanosomosis infections. Preferably, the bovine animal tested and the control bovine animal (or control bovine animal group) are of the same breed, and optionally of the same age and sex. The comparison of the quantities of aprotinin makes it possible to determine whether the bovine animal tested is tolerant or susceptible to trypanosomosis infections.

In the context of the present invention, determining the quantity of aprotinin present in the biological sample may be performed using any method known in the art suitable for protein quantification. In particular, aprotinin may be detected and quantified by means of immunoassay or immunological assay. A wide range of immunoassay techniques are available, such as radioimmunoassays, immunoenzyme assays, ELISA or solid substrate immunoenzyme assays, and immunofluorescence and immunoprecipitation assays. These assays are well known in the art and the methods for performing such assays have been described extensively.

In such embodiments, the method according to the invention comprises a step of contacting the biological sample with an anti-aprotinin antibody for a time and under conditions allowing the formation of an antibody-aprotinin complex between the antibody added and the aprotinin contained in the biological sample. Such anti-aprotinin antibodies are known in the art and some are commercially available (for example, anti-aprotinin antibodies with different epitopic specificities such as IgGlk clones 9A508, 9A509 and 9A511, USBiological, Euromedex). The method also uses a secondary antibody which binds to the immune complex and which is coupled to an enzyme, a chromogenic agent or a fluorogenic agent enabling detection. The quantity of aprotinin contained in the biological sample may be deduced from the quantity of complex formed.

The prognostic method according to the invention may be used to select the most tolerant animals in order to distribute the trypanotolerant characteristics throughout a herd. Those skilled in the art would appreciate that a prognosis of trypanotolerance may be performed solely on the basis of the results obtained in a method according to the invention. However, a veterinarian may also consider other parameters such as information provided by the livestock farmer relating to the health of the bovine animal under test during a trypanosomosis epidemic.

In an alternative embodiment of this second aspect, the present invention provides a kit for the prognosis of trypanosomosis infections in cattle. More specifically, the kit contains the materials required to perform a prognostic test according to the method of the invention. In general, a kit according to the invention comprises an antibody specific for aprotinin, a reagent for detecting an antibody-aprotinin complex formed between the antibody and the aprotinin contained in a biological sample (for example a secondary antibody detecting immune complexes) and instructions for carrying out the prognostic method according to the invention.

Depending on the procedure, the kit may further comprise extraction reagents or solutions, blocking reagents or solutions, labelling reagents or solutions, and/or detection means. Protocols for using these reagents and/or solutions may be included in the kit.

The various constituents of the kit may be provided in solid form (for example in freeze-dried form) or in liquid form. A kit may optionally comprise one or more vessels or containers containing each of the reagents or solutions, and/or one or more vessels or containers for carrying out certain steps of the prognostic method (test tubes, titration plates, etc.).

The instructions for carrying out a prognostic method according to the invention may include instructions for obtaining and processing the biological sample, instructions relating to the various steps of the prognostic method and/or instructions for interpreting the results. A kit according to the invention may also include a package insert in the form specified by a governmental agency regulating the preparation, sale and use of biological products.

Unless specified otherwise, all the technical and scientific terms used herein have the same meaning as that generally understood by a regular expert in the field of this invention.

The following examples and the figures are described to illustrate some embodiments of the procedures described above and should in no way be considered to be a limitation of the scope of the invention.

**Examples**

The inventors have conducted an overall transcriptome study using the Serial Analysis of Genes Expression (SAGE) technique on blood cells from trypanotolerant (N'Dama and Baoulé) and trypanosusceptible (Zebu) animals experimentally infected with *Trypanosoma congolense.* The infection kinetics study highlighted a number of over-expressed genes, including aprotinin. With respect to the transcripts of this gene, they observed an increase in number when the parasites were at their peak in the blood of trypanotolerant cattle, whereas they were practically non-detectable in the trypanosusceptible animal. The difference before infection and when the blood parasite level peaked was significant (P value $< 3. 10^{-4}$) and enabled the inventors to put forward the theory that the product of this gene could have an impact on, or be partly the result of, the trypanosome infection tolerance characteristic.

**Example 1: Determination of the effect of BPTI on macrophage NO**

Macrophages have a decisive role in the host response to protozoon infections. They are involved in the majority of inflammatory and immune responses. In the course of trypanosomosis infection, macrophages increase in number and size. Following macrophage activation, an induced enzyme, NO synthase, produces large quantities of nitrogen monoxide (NO). The purpose of this series of experiments is to functionally validate the ability of BPTI to induce NO production inhibition in mouse and bovine macrophages, *via* the action thereof on NO Synthase. The effects of BPTI on NO production were tested on two mouse macrophage lines (J774.2 and RAW 274.7) and one bovine macrophages derived from circulating monocytes.

***1. NO production inhibition by J774.2 macrophage line***

Two different vials of the J774.2 line were used at the 8th and 9th passages. The cells from these vials were placed in culture in parallel, and the tests were triplicated. The cells were first stimulated with Lipopolysaccharide (LPS, InvivoGen) at a concentration of 100 ng/ml and with mouse interferon gamma (IDNγ, AbD serotex) at a final concentration of 10 ng/ml and incubated in the absence (control) or in the presence of various concentrations of BPTI (range of 12.5 to 100 μM) for 15 hours at 37°C/5% $CO_2$ in a complete medium containing DMEM glutamax (Gibco) supplemented with 10% foetal calf serum (FCS) and 1% penicillin/streptamycin. The quantity of NO produced was determined by means of a colorimetric assay according to the Griess principle (Pinelli et al., Vet. Parasitol., 2000, 92: 181-189). For each BPTI concentration, NO production inhibition percentage is calculated according to the following formula:

$$\% \text{ inhibition} \quad = \quad 1 - \quad \frac{\text{NO concentration (in } \mu\text{M) for test sample}}{\text{mean NO concentration (in } \mu\text{M) for control}}$$

The results obtained, represented in graphs A and B in Figure 1, demonstrate the inhibitory action of BPTI on NO production by J774.2 macrophage line cells. This inhibition increases as a function of aprotinin concentration added and is proportional to this concentration. When the cells are stimulated, the mean quantity of NO produced was 83.2 $\mu$M. At BPTI concentrations of 12.5 $\mu$M, 25 $\mu$M, 50 $\mu$M and 100 $\mu$M, the inhibitions were 1.7%, 16.2%, 30.1% and 59.5% respectively.

### 2. NO production inhibition by RAW 264.7 macrophage line

Two different vials of the RAW 264.7 line were used at the 10th and 11th passages. The cells from these vials were placed in culture in parallel, and the tests were triplicated and performed as described above.

The results obtained, represented in graphs A and B in Figure 2, demonstrate the inhibitory action of BPTI on NO production by RAW 264.2 macrophage line cells. However, in this case, the inhibition is not linear as a function of BPTI concentration as was observed in the case of the J744.2 cells. In the stimulated control cells, the mean quantity of NO produced was 90.1 $\mu$M. At BPTI concentrations of 25 $\mu$M, 50 $\mu$M and 100 $\mu$M, the inhibitions were 0.2%, 16.1% and 47.6%, respectively. No inhibition was observed for the BPTI concentration of 12.5 $\mu$M.

### 3. Overall model for the effect of BPTI on NO production for both mouse cell lines

For each of the mouse cell lines, it was demonstrated that the cell passage number has no effect on the NO concentration (results shot shown). Similarly, a model based on the combined effect of the BPTI concentration and the cell line on the NO concentration demonstrated that this combined effect was not significant (results not shown). The following model was also used to study the inhibitory effects of BPTI on NO production and the effect of the cell line:

$$[NO]_i = [NO]_m + [BPTI]_i + cell_k + \varepsilon_{ik}$$

wherein, $[NO]_i$ is the NO concentration in $\mu$M observed in the cells incubated in the presence of BPTI at the concentration $[BPTI]_i$; $[NO]_m$ is the mean NO concentration in $\mu$M observed in the stimulated control cells receiving 12.5 $\mu$M of BPTI (the baseline value is estimated for 12.5 $\mu$M of BPTI due to a non-linear effect between 0 and 12.5 $\mu$M of BPTI for the RAW 264.7 line); $cell_k$ is the cell line of the cells in question (i.e. J774.2 or RAW 264.7); and $\varepsilon_{ik}$ represents the model residues assumed to observe a normal law $N(0,\sigma^2)$.

This model led to the results shown in the graph of Figure 3 which demonstrates that the estimated effect of the BPTI concentration on NO production inhibition is extremely significant (P-value < 10-15) and is independent of the cell line. The cell line has an effect only on the NO baseline level after LPS-INF$\gamma$ stimulation.

### 4. NO production inhibitions by bovine macrophages

Bovine monocytes were isolated from circulating monocytes. Two independent tests were performed and each of the tests was triplicated. The monocytes were stimulated with LPS at a concentration of 250 ng/ml and bovine IFNg at a final concentration of 10 ng/ml. The cells were then incubated in the absence (control) or in the presence of various concentrations of BPTI (12.5 to 100 $\mu$M) for 20 hours (test 1) and 24 hours (test 2) at 37°C/5% CO2 in IMDM complete medium containing IMDM supplemented with L-glutamine at 2 nM final, gentamicin at 50 $\mu$g/ml final, 10% FCS and 5x10$^{-5}$ M b-mercaptoethanol.

*a) Test 1:* The results obtained are represented in graphs A and B in Figure 4. They demonstrate the inhibitory action of BPTI on NO production by bovine macrophages. This inhibition increases as a function of concentration of BPTI present. In the stimulated control cells, the mean quantity of NO produced was 11.5 $\mu$M. At BPTI concentrations of 12.5 $\mu$M, 25 $\mu$M, 50 $\mu$M and 100 $\mu$M, the mean inhibitions induced by BPTI were 8.4%, 14.2%, 32.6% and 70.5%, respectively.

The results obtained were studied using the following model (see group C in Figure 4):

$$[NO]_i = [NO]_m + [BPTI]_i + \varepsilon_{ik}$$

wherein, $[NO]_i$ is the NO concentration in $\mu M$ observed in the cells incubated in the presence of BPTI at the concentration $[BPTI]_i$; $[NO]_m$ is the mean NO concentration in $\mu M$ observed in the stimulated control cells; and $\varepsilon_{ik}$ represents the model residues assumed to observe a normal law $N(0,\sigma^2)$. The effect of the BPTI concentration on NO nitric oxide production by bovine macrophages is extremely significant (P-value $< 2x10^{-16}$)

*Test 2*: The results obtained are represented in graphs A and B in Figure 5. Here again, an inhibitory effect of BPTI on NO production by bovine macrophages was observed. The inhibition increases as a function of concentration of BPTI present. In the stimulated control cells, the mean quantity of NO produced was 5.43 $\mu M$. At BPTI concentrations of 6.25 $\mu M$, 12.5 $\mu M$, 25 $\mu M$, 50 $\mu M$ and 100 $\mu M$, the mean inhibitions induced by BPTI were 12%, 25.9%, 62.5%, 70%, and 60.2%, respectively. In this test, the inhibition was only proportional for BPTI concentration between 6.25 and 50 $\mu M$. The results obtained cannot be studied with a simple model. They require the use of a polynomial model (see graph C in Figure 5).

### 5. Conclusion on NO production inhibition by BPTI

The BPTI molecule has an extremely significant inhibitory activity (P value $< 2.10^{-16}$) on NO produced by mouse macrophage lines but also on that of bovine macrophages obtained from circulating monocytes. In any case, a dose-dependent effect was observed. The most significant inhibitions were observed on bovine macrophages. BPTI is a bovine molecule and it is thus not surprising that its action is more effective on cells of the same species. Since BPTI is capable of inhibiting NO synthesis, it could induce alternative macrophage activation and block acute inflammatory processes.

### Example 2: Effects of BPTI on parasite viability and growth

### 1. Effect of BPTI on the viability of Trypanosoma congolense IL1180

a) *Microscopic observations:* The first microscopic observations revealed the effect of BPTI on parasite morphology (see Figure 6). Before adding BPTI and for measurement times: 1.5 hours, 17 hours and 24 hours, the parasites (*Trypanosoma congolense* IL1180) are clearly visible; their form is as expected (fusiform), and they are mobile. However, the mortality becomes increasingly visible over time. The impact of BPTI on morphology is perceptible 17 hours (and thereafter) after adding BPTI to the culture. Spherical forms are observed which are characterized by a state of stress of the parasites.

b) *Viability of Trypanosoma congolense IL1180 measured by FACS:* In order to analyze the effect of BPTI on parasite viability, flow cytometry measurements were made at different times (1.5 hours, 17 hours, 24 hours and 48 hours after adding 100 $\mu M$ or 200 $\mu M$ of BPTI for parasite concentrations of $5x10^6$ parasites/ml and $10x10^6$ parasites/ml). The results obtained are represented in graphs A and B in Figure 7.

There is a "natural" mortality of *Trypanosoma congolense in vitro,* due to the difficulty maintaining sanguicolous forms of *Trypanosoma congolense* in liquid culture. This mortality increases over time and reaches values of 49.1% and 47.1% under conditions using $5.10^6$ parasites/ml and $10.10^6$ parasites/ml, respectively. However, a trypanocidal action of BPTI may be observed on trypanosome cultures, a slight dose-dependent effect of BPTI on parasite viability may be recorded. The effect of BPTI is extremely significant and appears to be also time-dependent. Once the natural mortality has been subtracted, it is observed that BPTI concentrations of 100 $\mu M$ and 200 $\mu M$ induce 46% and 47.4% mortality, respectively, at 48 hours on a population of $2.5x10^6$ trypanosomes ($5x10^6$ parasites/ml). The mortality induced by BPTI on a population of $5x10^6$ trypanosomes ($10x10^6$ parasites/ml) under the same conditions is 49.6% and 48.8%, respectively (see graphs A and B in Figure 8).

c) *Statistical analyses of results obtained:* The inventors have modelled the effects of the BPTI concentration, time, trypanosome concentration and the test date on trypanosome mortality. Given that trypanosome mortality was measured over time in the same well and considering that a random variation of the trypanosome mortality between wells could occur, a composite linear model was used. This model detects the correlations which are due to the repetition of intra-well measurements, and which considers the wells to be distributed at random within a population to observe a normal law $N(0, \sigma_c^2)$.

The test of the effects of all the variables and possible interactions between these variables demonstrated that the "trypanosome concentration" effect and its various interactions are not significant. However, the measurement time has a significant effect on trypanosome mortality. This is not surprising since various cryostable trypanosomes, thawed at different times, were involved, thus possibly having specific viabilities based on the thawing conditions.

The interaction between the BPTI concentration and the measurement time (after adding BPTI) is very significant and positive (see Figure 9). Due to this interaction, the effect of BPTI for each measurement time values was studied. For all the conditions studied, it was found that the effect of BPTI on mortality was extremely significant (P value $<10^{-4}$) and increases over time.

### 2. Effect of BPTI on the viability of Leishmania donovani infantum (Ldi)

**a) Microscopic observations:** The growth and viability of *Leismania donovani infantum* parasites were measured by FACS at 4, 12, 24, 48, 72, 144 and 168 hours of culture after addition of BPTI at concentrations of 50, 100 and 200 $\mu$M. The results are presented in graphs A and B in Figure 10.

**b) Statistical analyses of the results obtained: Study of the effects of BPTI** on the viability of Leishmania: The graphic observation of the results (graph A in Figure 10) demonstrates a non-linear relationship between time and viability - viability increases up until 144 hours and then subsequently appears to decline. The effect of BPTI was tested for each time value with a non-parametric test (Kruskal-Wallis test). This test demonstrates a significant effect of BPTI on the viability of leishmania for the times 168 hours (P-value = 0.03) and 144 hours (P-value = 0.05) and at the limit of significance for 72 hours (P-value = 0.05); the effect was not significant for the other observation times.

*Study of the effects of BPTI on the growth of Leishmania:* Just like the viability of Leishmania, group B in Figure 10, which represents the number of leishmania (in log10) as a function of time, for different BPTI concentrations, demonstrates a non-linear relationship between leishmania population growth and the explanatory variables. When the effect BPTI for each time was tested with a non-parametric test (Kruskal-Wallis test), a significant effect of BPTI on the number of leishmania was demonstrated only for the time 168 hours (P-value = 0.03).

### 3. Conclusion on the effect of BPTI on parasite viability and growth

The results obtained demonstrate that BPTI has a trypanocidal and leishmanicidal action. For *Trypanosoma congolense,* the mortality was significant regardless of the measurement time (P-value < 10-4). For leishmania, the viability was only affected after a contact time of 72 hours. A significant action (P-value = 0.03) on leishmania growth was only observed after a contact time of 168 hours.

### Example 3: Effects of BPTI on parasite lysate enzyme activity

The molecules produced, excreted or secreted by the parasite have a significant impact on trypanosomosis diseases. Some molecules have already been identified as playing a major role in the condition. This is the case of parasite proteases such as congopain, a trypasomal cysteine protease having 33 kDa detected in the systemic circulation of cattle infected with *Trypanosoma congolese* (Authié et al., Int. J. Parasitol., 2001, 31: 1429-1433). It is also the case of Oligopeptidase B (OP-Tc), a trypanosomal serine protease also detected in the blood circulation (Morty et al., Mol. Biochem. Parasitol., 1999, 102: 145-155), but which, unlike congopain, is released in favor of parasite lysis induced by host responses. This enzyme is not degraded, it retains a significant catalytic activity detectable in the serum of infected animals (Troeberg et al., Eur. J. Biochem., 1996, 238: 728-736).
The purpose of this series of experiments is to determine whether BPTI can have an action on the enzyme activity of parasite lysates.

### 1. Effect of BPTI on Trypanosoma congolense (Tc. IL1180) lysates

The parasites were placed in rodent culture and purified on a DEAE cellulose column. The parasites were then counted, and lysed by means of 4 successive 1-minute passages in liquid nitrogen and in a water-bath at 37°C. The tests were conducted on three independent procedures in triplicate using $2 \times 10^{-6}$ parasites. The method used for detecting "trypsin-like" (serine protease) consists of measuring, as a function of time, the initial degradation rate of a homogeneous substrate, N-benzoyl-ariginine-p-nitroaniline (BAPNA), under the effect of "trypsin-like" found in lysates in the presence of absence of BPTI. The appearance of the degradation product, p-nitroalinine (p-Na), which is yellow in colour, is measured by means of spectrophotometry at 405 nm. The enzyme activity calculation is performed according to the Beer-Lambert law.
The graphs in Figure 11 represent the degradation kinetics of the substrate (BAPNA, 0.315 mM) in the presence or absence of BPTI (3.125 mg/ml or 481.25 $\mu$M or 625 $\mu$g/well). The initial lysate activity was 1.893 (Standard deviation 0.076) versus 1.048 (Standard deviation 0.073) in the presence of BPTI, *i.e.* a mean inhibition of 44.6%. The use of the Kruskal-Wallis non-parametric test demonstrated a very significant effect of BPTI on the enzyme activity of the parasite lysate (Kruskal-Wallis Chi Square: 14.5; Degree of freedom: 1; P value = 0.0001).

### 2. Effects of BPTI on Leishmania donovani infantum (Ldi) and Leishmania donovani donovani (Ldd8) lysates

Leishmania (Ldi and Ldd8) were placed in culture in RMPI supplemented with 10% FCS, 1% penicillin/streptomycin and 1% Ultraglutamine and placed in an incubator) 37°C/5% CO2 for one week. The leishmania are counted and lysed according to the protocol defined for trypanosome lysis. The method for detecting and the method for calculating the enzyme activity were identical to those used for trypanosomes. The tests using Ldi and Ldd8 were conducted on three independent procedures in triplicate using $2 \times 10^{-6}$ parasites.

*a) Inhibition of enzyme activity of Ldi lysate:* The results obtained are represented in the graphs in Figure 12. The activity in the lysate alone was 2.893 versus 1.555 with BPTI, *i.e.* an inhibition of activity of 46.3%. The use of the Krustal-Wallis non-parametric test demonstrated a very significant effect of BPTI on the enzyme activity of the parasite lysate (Kruskal-Wallis Chi Square: 12.91; Degree of freedom: 1; p value= 0.0003).

*b) Inhibition of enzyme activity of Ldd8 lysate:* The results obtained are represented in the graphs in Figure 13. The activity in the lysate alone was 1.767, versus 1.0893 with BPTI *i.e.* an inhibition of activity of 49,5%. The use of the Krustal-Wallis non-parametric test demonstrated a very significant effect of BPTI on the enzyme activity of the parasite lysate (Kruskal-Wallis Chi Square: 13.37; Degree of freedom: 1; p value=0.0003).

### 3. Conclusion on the effect of BPTI on parasite lysate enzyme activity

A decrease in enzyme activity was observed in all the parasite lysates used. BPTI induced a significant reduction of 44.6%, 46.3% and 49.5%, respectively, of the enzyme activity of *Trypanosoma congolense* (P value 0.0001), *Leishmania donovani donovani* (P value 0.0003) and *Leishmania donovani infantum* (P value 0.0002). The action of BPTI on the lysate enzyme activity of these parasites is thus well-established.

Proteases are significant for parasites (McKerrow et al., Annu. Rev. Pathol., 2006, 1: 497-536). Their functions and roles are varied. In trypanosomes, the main proteolytic enzymes are cysteine proteases and serine proteases (Mbawa et al., Eur. J. Biochem., 1992, 204: 371-379; Troeberg et al., Eur. J. Biochem., 1996, 238: 728-736). As mentioned above, the role of these enzymes in the severity of the disease has frequently been suggested (Boulange et al., Int. J. Parasitol., 2001, 31: 1435-1440; Authie et al., Int. J. Parasitol., 2001, 31: 1429-1433; Authié, Parasitol. Today, 1994, 10: 360-364; Authié et al., Mol. Biochem. Parasitol., 1992, 56: 103-116).

The results obtained demonstrate that BPTI could counteract the pathogenic effects of parasite proteases in cases of infection.

### Other Embodiments

Other embodiments of the invention will be apparent to those skilled in the art from a consideration of the specification or practice of the invention disclosed herein. It is intended that the specification and examples be considered as exemplary only, with the true scope of the invention being indicated by the following claims.

### Claims

1. Aprotinin for use in the treatment of an animal parasitic disease or infection that is caused by a flagellate protozoan of the *Trypanosomatidae* family and that affects mammals.

2. Aprotinin according to claim 1, wherein said animal parasitic disease or infection affects domesticated mammals selected from the group consisting of cattle, goats, sheep, and buffalo.

3. Aprotinin according to claim 1 or claim 2, wherein the animal parasitic disease or infection is a trypanosomosis and is caused by a flagellate protozoan of the *Trypanosoma* genus.

4. Aprotinin according to claim 3, wherein the animal parasitic disease or infection is caused by a trypanosome selected from the group consisting of *Trypanosoma congolense, Trypanosoma brucei* and *Trypanosoma vivax.*

**5.** Aprotinin according to claim 3 or claim 4, wherein the animal parasitic disease or infection is a bovine trypanosomosis.

**6.** Aprotinin according to claim 1 or claim 2, wherein the animal parasitic disease or infection is a leishamaniasis and is caused by a flagellate protozoan of the *Leishmania* genus.

**7.** Aprotinin according to claim 6, wherein the animal parasitic disease or infection is caused by a leishmania selected from the group consisting of *Leishmania donovani donovani* and *Leishmania donovani infantum.*

**8.** Pharmaceutical composition comprising aprotinin and at least one acceptable veterinary vehicle or excipient for use in the treatment of an animal parasitic disease or infection caused by a flagellate protozoan of the *Trypanosonzatidae* family.

**9.** A pharmaceutical composition for the use according to claim 8, wherein said animal parasitic disease or infection affects domesticated mammals selected from the group consisting of cattle, goats, sheep, and buffalo.

**10.** A pharmaceutical composition for the use according to claim 8 or claim 9, wherein the animal parasitic disease or infection is a trypanosomosis and is caused by a flagellate protozoan of the *Trypanosoma* genus.

**11.** A pharmaceutical composition for the use according to claim 10, wherein the animal parasitic disease or infection is caused by a trypanosome selected from the group consisting of *Trypanosoma congolense, Trypanosoma brucei* and *Trypanosoma vivax.*

**12.** A pharmaceutical composition for the use according to claim 10 or claim 11, wherein the animal parasitic disease or infection is a bovine trypanosomosis.

**13.** A pharmaceutical composition for the use according to claim 8 or claim 9, wherein the parasitic disease or infection is a leishmaniasis and is caused by a flagellate protozoan of the *Leishmania* genus.

**14.** A pharmaceutical composition for the use according to claim 13, wherein the animal parasitic disease or infection is caused by a leishmania selected from the group consisting of *Leishmania donovani donovani* and *Leishmania donovani infantum.*

**15.** A pharmaceutical composition for the use according to any one of claims 8 to 14, further comprising at least one pharmaceutically active agent.

**16.** An *in vitro* method for the prognosis of a trypanosomosis infection in a bovine, the method comprising a step of:

determining the quantity of aprotinin in a biological sample obtained from the bovine.

**17.** An *in vitro* method according to claim 16, wherein the step of determining the quantity of aprotinin comprises using an immunological assay method, particularly an ELISA test.

**18.** An *in vitro* method according to claim 16 or claim 17, wherein the biological sample is blood or serum.

**Patentansprüche**

**1.** Aprotinin zur Verwendung in der Behandlung einer parasitären Tiererkrankung oder -infektion, die durch ein flagelliertes Protozoon der Familie *Trypanosomatidae* verursacht wird und die Säugetiere betrifft.

**2.** Aprotinin nach Anspruch 1, wobei die parasitäre Tiererkrankung oder -infektion domestizierte Säugetiere, ausgewählt aus der Gruppe, bestehend aus Rindern, Ziegen, Schafen und Büffeln, betrifft.

**3.** Aprotinin nach Anspruch 1 oder Anspruch 2, wobei die parasitäre Tiererkrankung oder -infektion eine Trypanosomiasis ist und durch ein flagelliertes Protozoon des Genus *Trypanosoma* verursacht wird.

**4.** Aprotinin nach Anspruch 3, wobei die parasitäre Tiererkrankung oder -infektion durch ein Trypanosoma, ausgewählt aus der Gruppe, bestehend aus *Trypanosoma congolese, Trypanosoma brucei* und *Trypanosoma vivax,* verursacht wird.

**5.** Aprotinin nach Anspruch 3 oder Anspruch 4, wobei die parasitäre Tiererkrankung oder -infektion eine bovine Trypanosomiasis ist.

**6.** Aprotinin nach Anspruch 1 oder Anspruch 2, wobei die parasitäre Tiererkrankung oder -infektion eine Leishmaniose ist und durch ein flagelliertes Protozoon des Genus *Leishmania* verursacht wird.

**7.** Aprotinin nach Anspruch 6, wobei die parasitäre Tiererkrankung oder -infektion durch eine Leishmanie, ausgewählt aus der Gruppe, bestehend aus *Leishmania donovani donovani* und *Leishmania donovani infantum,* verursacht wird.

**8.** Pharmazeutische Zusammensetzung umfassend Aprotinin und mindestens ein annehmbares veterinäres Vehikel oder Hilfsmittel zur Verwendung in der Behandlung einer parasitären Tiererkrankung oder -infektion, die durch ein flagelliertes Protozoon der Familie *Trypanosomatidae* verursacht wird.

**9.** Pharmazeutische Zusammensetzung zur Verwendung nach Anspruch 8, wobei die parasitäre Tiererkrankung oder -infektion domestizierte Säugetiere, ausgewählt aus der Gruppe, bestehend aus Rindern, Ziegen, Schafen und Büffeln, betrifft.

**10.** Pharmazeutische Zusammensetzung zur Verwendung nach Anspruch 8 oder Anspruch 9, wobei die parasitäre Tiererkrankung oder -infektion eine Trypanosomiasis ist und durch ein flagelliertes Protozoon des Genus *Trypanosoma* verursacht wird.

**11.** Pharmazeutische Zusammensetzung zur Verwendung nach Anspruch 10, wobei die parasitäre Tiererkrankung oder -infektion durch ein Trypanosoma, ausgewählt aus der Gruppe, bestehend aus *Trypanosoma congolese, Trypanosoma brucei* und *Trypanosoma vivax,* verursacht wird.

**12.** Pharmazeutische Zusammensetzung zur Verwendung nach Anspruch 10 oder Anspruch 11, wobei die parasitäre Tiererkrankung oder -infektion eine bovine Trypanosomiasis ist.

**13.** Pharmazeutische Zusammensetzung zur Verwendung nach Anspruch 8 oder Anspruch 9, wobei die parasitäre Erkrankung oder Infektion eine Leishmaniose ist und durch ein flagelliertes Protozoon des Genus *Leishmania* verursacht wird.

**14.** Pharmazeutische Zusammensetzung zur Verwendung nach Anspruch 13, wobei die parasitäre Tiererkrankung oder -infektion durch eine Leishmanie, ausgewählt aus der Gruppe, bestehend aus *Leishmania donovani donovani* und *Leishmania donovani infantum,* verursacht wird.

**15.** Pharmazeutische Zusammensetzung zur Verwendung nach einem der Ansprüche 8 bis 14, weiterhin umfassend mindestens einen pharmazeutischen Wirkstoff.

**16.** *In vitro* Verfahren zur Prognose einer Trypanosomiasis-Infektion in einem Rind, wobei das Verfahren einen Schritt umfasst von: Bestimmen der Menge an Aprotinin in einer biologischen Probe, die aus dem Rind erhalten wird.

**17.** *In vitro* Verfahren nach Anspruch 16, wobei der Schritt des Bestimmens der Menge an Aprotinin Verwendung eines immunologischen Testverfahrens umfasst, insbesondere eines ELISA-Tests.

**18.** *In vitro* Verfahren nach Anspruch 16 oder Anspruch 17, wobei die biologische Probe Blut oder Serum ist.

**Revendications**

**1.** Aprotinine pour une utilisation dans le traitement d'une maladie ou d'une infection parasitaire animale qui est provoquée par un protozoaire flagellé de la famille des *Trypanosomatidae* et qui affecte les mammifères.

**2.** Aprotinine selon la revendication 1, où ladite maladie ou infection parasitaire animale affecte des mammifères domestiqués choisis dans le groupe constitué des bovins, des chèvres, des moutons, et des buffles.

**3.** Aprotinine selon la revendication 1 ou la revendication 2, où la maladie ou infection parasitaire animale est une trypanosomiase et est provoquée par un protozoaire flagellé du genre *Trypanosoma*.

**4.** Aprotinine selon la revendication 3, où la maladie ou infection parasitaire animale est provoquée par un trypanosome choisi dans le groupe constitué de *Trypanosoma congolense, Trypanosoma brucei* et *Trypanosoma vivax*.

**5.** Aprotinine selon la revendication 3 ou la revendication 4, où la maladie ou infection parasitaire animale est une trypanosomiase bovine.

**6.** Aprotinine selon la revendication 1 ou la revendication 2, où la maladie ou infection parasitaire animale est une leishmaniose et est provoquée par un protozoaire flagellé du genre *Leishmania.*

**7.** Aprotinine selon la revendication 6, où la maladie ou infection parasitaire animale est provoquée par une Leishmania choisie dans le groupe constitué de *Leishmania donovani donovani* et *Leishmania donovani infantum*.

**8.** Composition pharmaceutique comprenant de l'aprotinine et au moins un véhicule ou un excipient vétérinaire acceptable pour une utilisation dans le traitement d'une maladie ou d'une infection parasitaire animale provoquée par un protozoaire flagellé de la famille des *Trypanosomatidae*.

**9.** Composition pharmaceutique pour une utilisation selon la revendication 8, où ladite maladie ou infection parasitaire animale affecte des mammifères domestiqués choisis dans le groupe constitué des bovins, des chèvres, des moutons, et des buffles.

**10.** Composition pharmaceutique pour une utilisation selon la revendication 8 ou la revendication 9, où la maladie ou infection parasitaire animale est une trypanosomiase et est provoquée par un protozoaire flagellé du genre *Trypanosome.*

**11.** Composition pharmaceutique pour une utilisation selon la revendication 10, où la maladie ou infection parasitaire animale est provoquée par un trypanosome choisi dans le groupe constitué de *Trypanosoma congolaise, Trypanosoma brucei* et *Trypanosoma vivax.*

**12.** Composition pharmaceutique pour une utilisation selon la revendication 10 ou la revendication 11, où la maladie ou infection parasitaire animale est une trypanosomiase bovine.

**13.** Composition pharmaceutique pour une utilisation selon la revendication 8 ou la revendication 9, où la maladie ou infection parasitaire est une leishmaniose et est provoquée par un protozoaire flagellé du genre *Leishmania.*

**14.** Composition pharmaceutique pour une utilisation selon la revendication 13, où la maladie ou infection parasitaire animale est provoquée par une Leishmania choisie dans le groupe constitué de *Leishmania donovani donovani* et *Leishmania donovani infantum.*

**15.** Composition pharmaceutique pour une utilisation selon l'une quelconque des revendications 8 à 14, comprenant en outre au moins un agent pharmaceutiquement actif.

**16.** Procédé *in vitro* pour le pronostic d'une infection de trypanosomiase chez un bovin, le procédé comprenant une étape de :

détermination de la quantité d'aprotinine dans un échantillon biologique obtenu à partir du bovin.

**17.** Procédé *in vitro* selon la revendication 16, dans lequel l'étape de détermination de la quantité d'aprotinine comprend l'utilisation d'un procédé de test immunologique, en particulier un test ELISA.

**18.** Procédé *in vitro* selon la revendication 16 ou la revendication 17, dans lequel l'échantillon biologique est du sang ou du sérum.

Figure 1 / 13

**A**

NaNO2 concentration(μM) depending on [BPTI]

μM NaNO2

Stimulation control — 12,5 — 25 — 50 — 100

μM BPTI

**B**

NO inhibition (%) depending on [BPTI]

% INHIB

12,5 — 25 — μM BPTI — 50 — 100

Figure 2 / 13

**A**

NaNO2 concentration (µM) depending on [BPTI]

**B**

NO inhibition (%) depending on [BPTI]

**Figure 3 / 13**

## Figure 4 / 13

**A**

NaNO2 concentration (µM) depending on [BPTI]; Test 1

**B**

NO inhibition (%) depending on [BPTI]; Test 1

**C**

## Figure 5 / 13

**A**

NaNO2 concentration (µM) depending on [BPTI]; Test 2

**B**

NO inhibition (%) depending on [BPTI]; Test 2

**C**

Figure 6 / 13

A

a. without BPTI

B

b. with BPTI (200 μM)

Zoom

Zoom

Figure 7 / 13

**A**

BPTI effect on IL1180 viability (5.10⁶/ml)

**B**

BPTI effect on IL1180 viability (10.10⁶/ml)

Figure 8 / 13

**A**

Trypanosoma congolense IL1180 [5.10$^5$ Tryp/ml] viability under a BPTI treatment

**B**

Trypanosoma congolense IL1180 [10.10$^5$ Tryp/ml] viability under a BPTI treatment

## Figure 9 / 13

## Figure 10 / 13

**A**

**B**

## Figure 11 / 13

**A**

Kinetic curve of BAPNA degradation by trypanosomes Tc. IL1180 lysates (2.10⁶ parasites) with or without BPTI

**B**

Enzymatic activity on trypanosomes Tc.IL1180 lysates

**C**

Enzymatic activity of trypanosomes Tc. IL1180 lysates with or without BPTI

## Figure 12 / 13

**A**

Kinetic curve of BAPNA degradation by leishmanies Ldi lysates (2.10[6] parasites) with or without BPTI

**B**

Enzymatic activity of Leishmanies Ldi lysates

**C**

Enzymatic activity of leishmanies Ldi lysates with or without BPTI

## Figure 13 / 13

**A**

Kinetic curve of BAPNA degradation by leishmanies Ldd8 lysates (2.10⁶ parasites) with or without BPTI

**B**

Enzymatic activity of Leishmanies Ldd8 lysates

**C**

Enzymatic activity of leishmanies Ldd8 lysates with or without BPTI

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KASSEL et al.** *Biochem. Biophys. Res. Commun.,* 1965, vol. 18, 255-258
- **KASSEL et al.** *Biochem. Biophys. Res. Commun.,* 1965, vol. 20, 463-468
- **VAN OEVEREN et al.** *Ann. Thorac. Surg.,* 1987, vol. 44, 640-645
- **BISTRUP et al.** *Lancet,* 1988, vol. I, 366-367
- **ROYSTON.** *J. Cardiothorac. Vasc. Anesth.,* 1992, vol. 6, 76-100
- **PORTE et al.** *J. Cardiothorac. Vasc. Anesth.,* 2004, vol. 18 (4), 31 S-37S
- **ASIMAKOPOULOS et al.** *J. Thorac. Cardiovasc. Surg.,* 2000, vol. 120 (2), 361-369
- **MOJCIK et al.** *Ann. Thorac. Surg.,* 2001, vol. 71 (2), 745-754
- **ASCENZI et al.** *Curr. Protein Pept. Sci.,* 2003, vol. 4 (3), 231-251
- **BRUDA et al.** *Clin. Sci. (London),* 1998, vol. 94, 505-509
- **VENTURI et al.** *Biochem. Biophys. Res. Commun.,* 1998, vol. 249, 263-265
- **PINELLI et al.** *Vet. Parasitol.,* 2000, vol. 92, 181-189
- **AUTHIÉ et al.** *Int. J. Parasitol.,* 2001, vol. 31, 1429-1433
- **MORTY et al.** *Mol. Biochem. Parasitol.,* 1999, vol. 102, 145-155
- **TROEBERG et al.** *Eur. J. Biochem.,* 1996, vol. 238, 728-736
- **MCKERROW et al.** *Annu. Rev. Pathol.,* 2006, vol. 1, 497-536
- **MBAWA et al.** *Eur. J. Biochem.,* 1992, vol. 204, 371-379
- **BOULANGE et al.** *Int. J. Parasitol.,* 2001, vol. 31, 1435-1440
- **AUTHIE et al.** *Int. J. Parasitol.,* vol. 31, 1429-1433
- **AUTHIÉ.** *Parasitol. Today,* 1994, vol. 10, 360-364
- **AUTHIÉ et al.** *Mol. Biochem. Parasitol.,* 1992, vol. 56, 103-116